(19) Europäisches Patentamt
European Patent Office
Office européen des brevets

(11) Veröffentlichungsnummer: **0 102 531**
**B1**

(12) # EUROPÄISCHE PATENTSCHRIFT

(45) Veröffentlichungstag der Patentschrift: **02.03.88**

(51) Int. Cl.$^4$: **C 07 C 175/00,** C 07 C 131/10

(21) Anmeldenummer: **83107683.1**

(22) Anmeldetag: **04.08.83**

(54) **Verfahren zur Herstellung von Verbindungen der Megastigman-Reihe sowie neue Zwischenprodukte für diese Synthese.**

(30) Priorität: **21.08.82 DE 3231189**

(43) Veröffentlichungstag der Anmeldung:
**14.03.84 Patentblatt 84/11**

(45) Bekanntmachung des Hinweises auf die Patenterteilung:
**02.03.88 Patentblatt 88/09**

(84) Benannte Vertragsstaaten:
**CH DE FR GB LI NL**

(58) Entgegenhaltungen:
**EP-A-0 075 237**
**FR-A-2 371 412**
**FR-A-2 385 675**

(73) Patentinhaber: **BASF Aktiengesellschaft**
**Carl-Bosch-Strasse 38**
**D-6700 Ludwigshafen (DE)**

(72) Erfinder: **Janitschke, Lothar, Dr.**
**Wormser Gasse 9a**
**D-6711 Kleinniedesheim (DE)**
Erfinder: **Hoffmann, Werner, Dr.**
**Ringstrasse 11 c**
**D-6708 Neuhofen (DE)**

**EP 0 102 531 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Verfahren zur Herstellung von Verbindungen der Megastigman-Reihe ausgehend von dem neuen 1-Formyl-2,6,6-trimethyl-3-oximido-cyclohex-1-en (II) über die neuen Alkohole der allgemeinen Formel III

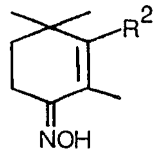

(III),

in der $R^2$ für einen der Reste

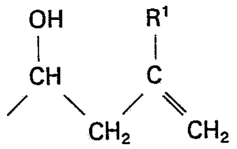 ; 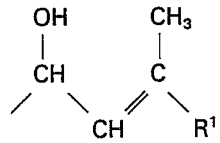 ;

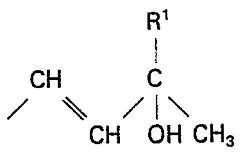 oder 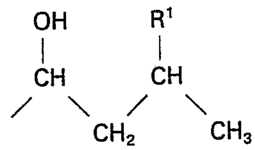

steht, sowie 3-(3-Oxo-but-1-en-1-yl)-2,4,4-trimethylcyclohex-2-en-1-on-oxim (VII) und 3-(1-Hydroxy-but-2-in-1-yl)-2,4,4-trimethyl-cyclohex-2-en-1-on-oxim (VIII) als neue Zwischenprodukte und 3-(3-Methyl-buta-1,3-dien-1-yl)-2,4,4-trimethyl-cyclohex-2-en-1-on als neuen Riechstoff der Megastigman-Reihe.

Megastigma-5,7(E),9-trien-4-on (Ia) und Megastigma-5,8(E)-dien-4-on (IV) sind Inhaltsstoffe von Osmanthus Absolues (vgl. Helv. Chim. Acta *61* (1978) Seiten 2328—35). IV und Megastigma-5,8(Z)-dien-4-on (V) sind Aromastoffe der gelben Passionsfrucht (vgl. Helv. Chim. Acta *62* (1979) seiten 67—75).

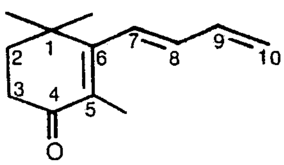

Ia

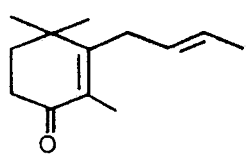

IV

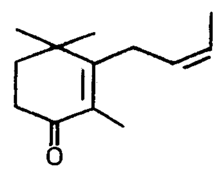

V

Verbindungen dieses Typs haben als Riechstoffe sowie als Geschmackstoffkompositionen großes Interesse gefunden.

So werden in der DE—A—27 52 787 Verbindungen der allgemeinen Formel (VI)

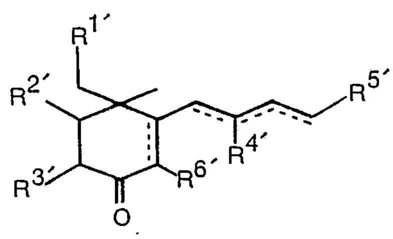

(VI),

in der $R^{1'}$ bis $R^{5'}$ für H oder $-CH_3$ stehen und $R^{6'}$ H, $-CH_3$, $-C_2H_5$, $=CH_2$ oder $=CH-CH_3$ bedeuten kann und in der $R^{4'}$ und $R^{5'}$ tragenden Seitenkette nur 1 C—C-Doppelbindung enthalten ist, wegen ihres stark frucht-ähnlichen Geschmacks und ihres angenehmen Duftes mit holz- und kräuterähnlichen Noten als Verbindungen mit wertvollen organoleptische Eigenschaften bezeichnet.

Folgende Herstellungsmöglichkeiten werden beschrieben:
A. ausgehend von β- oder γ-Damascon:

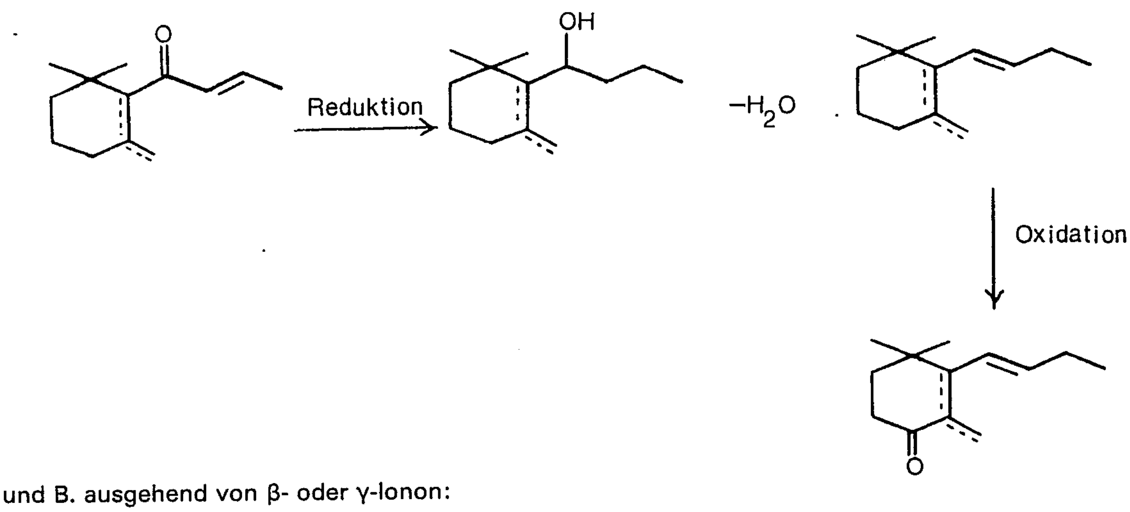


und B. ausgehend von β- oder γ-Ionon:

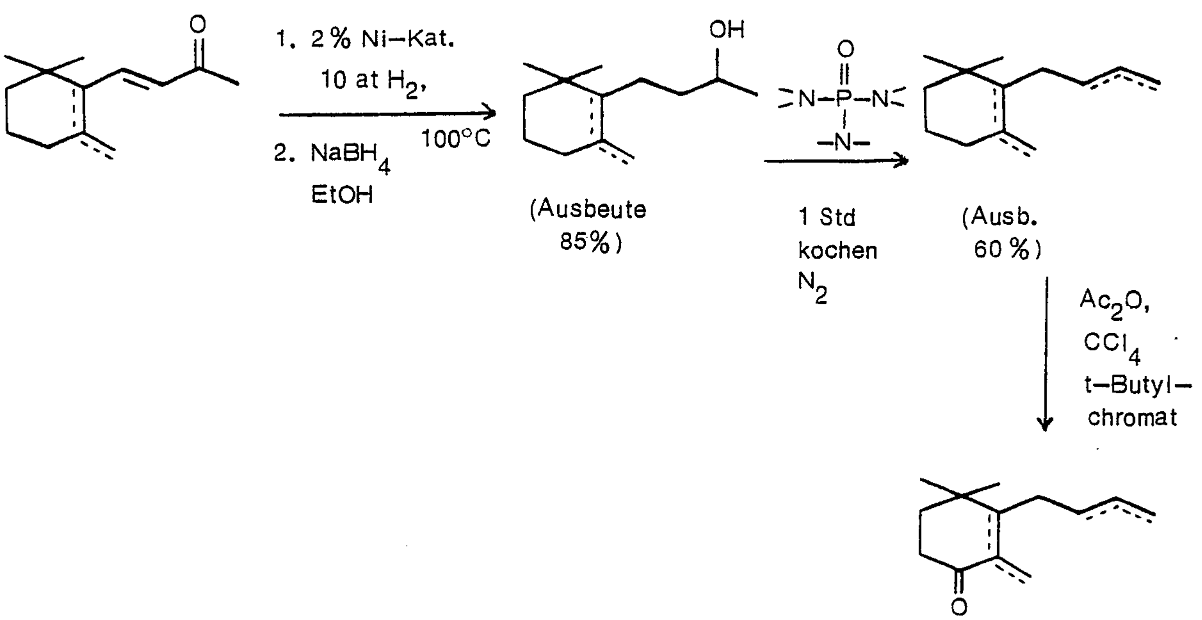


Für die letzte Stufe von B sind keine Ausbeuteangaben gemacht. Es heizßt nur, daß sich die Verbindungen durch präparative Gaschromatographie trennen ließen.

Zum Syntheseweg A werden keine praktischen Beispiele beschrieben. Bei der Bewertung dieses Reaktionsweges ist jedoch zu beachten, daß die als Ausgangsstoffe verwendeten Damascone, besonders das γ-Damascon, bereits sehr wertvolle Verbindungen sind, deren Herstellung über mehrere Stufen erfolgt. Selbst wenn die Ausbeuten denen gemäß Weg B vergleichbar wären, wäre dieser Weg weniger attraktiv als dieser.

Aus der DE—A—28 12 583 ist die Herstellung von wertvollen Riech- und Geschmackstoffen der Formel

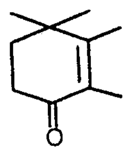

in der R für Butyl, But-2-en-1-yl oder 1,3-Butadienyl stehen, bekannt. Für die Synthese sind folgende Wege angegeben:

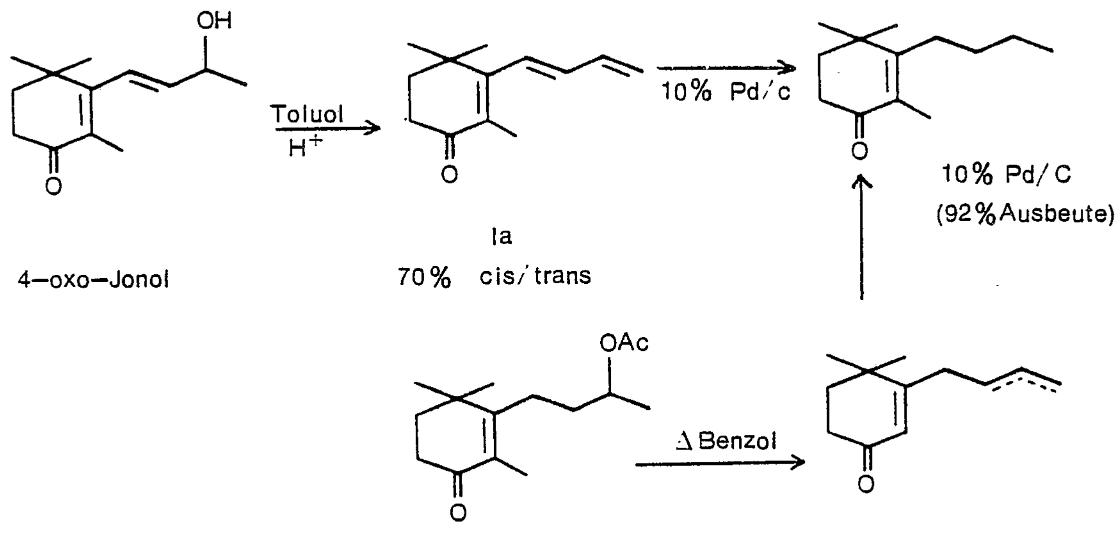


4—oxo—Jonol

Ia

70% cis/trans

4—oxo—dihydro—β—Jonol—Acetat

(ca. 54% Ausbeute; cis/trans—Gemisch)

Auch aus den eingangs zitierten Literaturstellen in Helv. Chim. Acta sind Synthesen der Verbindungen IV und V bekannt. Allen Verfahren gemeinsam ist, daß die Ausbeuten an den gewünschten Verbindungen relativ gering sind und daß sie über viele teils sehr aufwendige Reaktionsstufen führen oder aber die Ausgangsverbindungen nur über vielstufige Synthesewege zugänglich sind.

Es war daher die Aufgabe der Erfindung, ein Verfahren zu entwickeln, nach dem die Verbindungen der allgemeinen Formel I auf einfache Weise und aus relativ einfach zugänglichen Verbindungen herzustellen sind.

Es wurde nun gefunden, daß man die Verbindungen der Formel I ausgehend von dem neuen 1-Formyl-2,6,6-trimethyl-3-oximido-cyclohex-1-en der Formel II über die neuen Alkohole der allgemeinen Formel III in überraschend guten Gesamtausbeuten herstellen kann. Da II gemäß dem Verfahren der nichtvervöffentlichten EP—A—75 237 (P 31 37 802.1) auf überraschend einfache Weise aus Cyclocitral in hoher Ausbeute hergestellt werden kann, ergibt sich ein sehr vorteilhaftes Verfahren zur Herstellung der gewünschten Riech- und Aromastoffe.

Gegenstand der Erfindung ist deshalb ein Verfahren zur Herstellung von Verbindungen der Megastigman-Reihe der allgemeinen Formel I

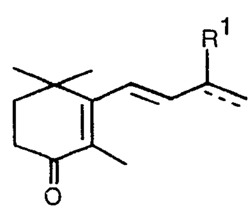

(I),

in der $R^1$ für Wasserstoff oder Methyl steht und die gestrichelte Linie für eine weitere C—C-Bindung stehen kann, das dadurch gekennzeichnet ist, daß man

A) das neue 1-Formyl-2,6,6-trimethyl-3-oximido-cyclohex-1-en der Formel II

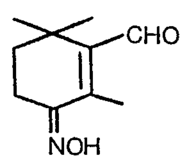

(II)

in an sich bekannter Weise durch Grignardreaktion mit Allylmagnesiumchlorid, Allylmagnesiumbromid, Propylmagnesiumchlorid oder Propylmagnesiumbromid oder durch Grignardreaktion mit Propinylmagnesiumchlorid oder Propinylmagnesiumbromid und anschließend Partialhydrierung der Dreifachbindung der Seitenkette oder aber durch Umsetzen mit Aceton in Gegenwart alkalischer Katalysatoren und

anschließende Reduktion der Carbonylgruppe der Seitenkette zur Hydroxylgruppe oder anschließende Grignardreaktion mit Methylmagnesiumchlorid oder Methylmagnesiumbromid in einen neuen Alkohol der allgemeinen Formel III

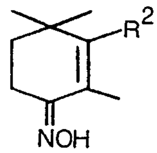

(III),

in der $R^2$ für einen der Reste

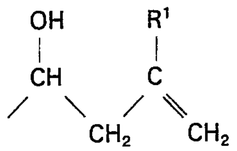 ; 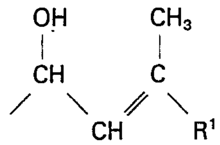 ;

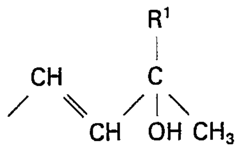 oder 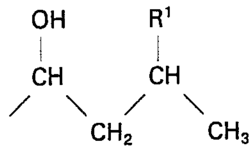

steht, überführt und

B) den erhaltenen Alkohol der Formel III in Gegenwart von Wasser und einer schwerflüchtigen starken Säuren auf Temperaturen von 90 bis 120, vorzugsweise 100 bis 115 erhitzt.

Zur Herstellung von Verbindungen der allgemeinen Formel I

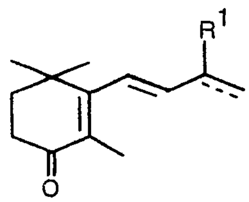

(I),

in der $R^1$ für H steht und die gestrichelte Linie eine weitere C—C-Bindung bedeutet, gemäß diesem Verfahren geht man so vor, daß man im Reaktionsschritt A

a) das 1-Formel-2,6,6-trimethyl-3-oximido-cyclohex-1-en der Formel II durch Grignardreaktion mit Allylmagnesiumchlorid oder Allylmagnesiumbromid in den Alkohol der Formel IIIa

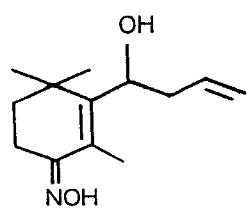

(IIIa)

überführt oder

b) II durch Grignard-Reaktion mit Propinylmagnesiumchlorid oder Propinylmagnesiumbromid und anschließende Partialhydrierung der Dreifachbindung der Seitenkette in den Alkohol der Formel IIIb

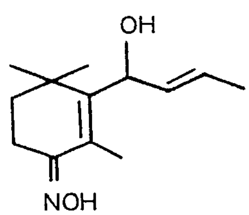

(IIIb)

überführt oder aber

c) II durch Umsetzen mit Aceton in Gegenwart alkalischer Katalysatoren zunächst in das neue Keton der Formel VII

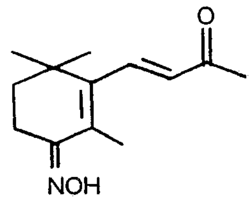

(VII)

und durch anschließende Reduktion der Carbonylgruppe der Seitenkette zur Hydroxylgruppe in den Alkohol der Formel IIIc

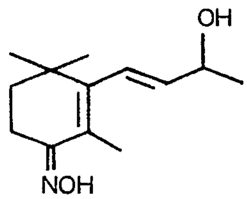

(IIIc)

überführt.

Zur Herstellung der Verbindung der allgemeinen Formel I

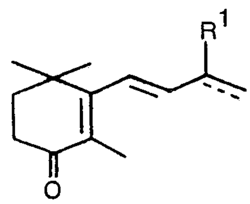

(I),

in der $R^1$ für H steht und die gestrichelte Linie keine Bedeutung hat, gemäß dem erfindungsgemäßen Verfahren geht man so vor, daß man im Reaktionsschritt A das 1-Formyl-2,6,6-trimethyl-3-oximido-cyclohex-1-en der Formel II durch Grignard-Reaktion mit Propylmagnesiumchlorid oder Propylmagnesiumbromid in den Alkohol der Formel IIId,

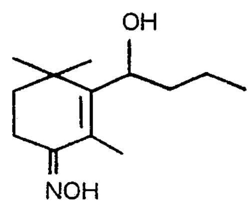

(IIId)

überführt.

Zur Herstellung der Verbindung der allgemeinen Formel I

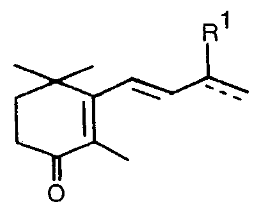

(I),

in der $R^1$ für $—CH_3$ steht und die gestrichelte Linie eine C—C-Bindung bedeutet, geht man so vor, daß man im Reaktionsschritt A das 1-Formyl-2,6,6-trimethyl-3-oximido-cyclohex-1-en der Formel II durch Umsetzen mit Aceton in Gegenwart alkalischer Katalysatoren zunächst in das neue Keton der Formel VII

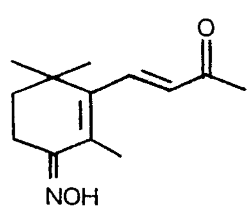

(VII)

und durch anschließende Grignardreaktion mit Methylmagnesiumchlorid oder Methylmagnesiumbromid in den Alkohol der Formel IIIe überführt.

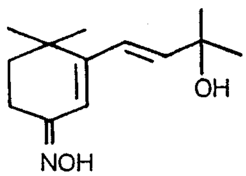
(IIIe)

Das erfindungsgemäße Verfahren eröffnet einen neuen Syntheseweg, gemäß dem man beispielsweise Megastigma-5,7,9-trien-4-on in nur 4 Stufen ausgehend von Citral über Cyclocitral, II und den Alkohol IIIa in einer Ausbeute über den gesamten Syntheseweg von 45 bis 70% der Theorie erhalten kann. Zu der guten Gesamtausbeute tragen besonders die hohen Ausbeuten im Oxidationsschritt, die überraschend hohe Selektivität bei der Grignard-Reaktion sowie die sehr guten Ausbeuten bei der gleichzeitigen Oximspaltung und Wasserabspaltung bei.

Zur Überführung von 1-Formyl-2,6,6-trimethyl-3-oximido-cyclo-hex-1-en (II) in den Alkohol IIIa setzt man II vorteilhaft mit der Lösung eines Allylmagnesiumhalogenids, insbesondere Allylmagnesiumchlorid, in einem etherischen Lösungsmittel wie Tetrahydrofuran (THF) um und arbeitet das Reaktionsgemisch in einer für Grignard-Reaktionen üblichen Weise hydrolytisch auf. Wegen des aktiven Wasserstoffs in der Oximgruppe ist für diese Umsetzung mindestens ein molarer Überschuß an dem Allylmagnesiumhalogenid nötig. Im allgemeinen arbeitet man mit Allylmagnesiumhalogenidmengen von 2 bis 3 mol pro mol II. Die Grignardreaktion verläuft mit hoher Selektivität an der Formylgruppe. Man erhält den Alkohol IIIa in Ausbeuten von 85 bis 93% der Theorie.

Zur gleichzeitigen Abspaltung der Oximgruppe und Wasser aus den Alkoholen der Formel III unter Bildung der Verbindungen der Formel I werden die Alkohole in Gegenwart von Wasser und einer schwerflüchtigen starken Säure auf Temperaturen von 90 bis 120°C, insbesondere 100 bis 115°C erhitzt. Als starke schwerflüchtige Säuren verstehen wir erfindungsgemäß Säuren mit einem $pK_s$-Wert kleiner als 2,5, die nicht oxydierend oder reduzierend auf die Alkohole der Formel III einwirken können und die nicht Ausbeuteverluste durch Angriff auf die Doppelbindungen in den Alkoholen der Formel III hervorrufen können. Genannt seien beispielsweise Schwefelsäure, Phosphorsäure, p-Toluolsulfonsäure, Trichloressigsäure und stark saure Ionenaustauscher. Mit besonderem Vorteil verwendet man wäßrige Schwefelsäure, und zwar in Form einer 5 bis 50 gew.%igen, vorzugsweise 10 bis 40 gew.%igen, insbesondere einer etwa 20 bis 35 gew.%igen wäßrigen Schwefelsäure.

Zur Überführung von II in den Alkohol IIIb setzt man II vorteilhaft mit der Lösung eines Propinylmagnesiumhalogenids, insbesondere Propinylmagnesiumchlorid oder Propinylmagnesiumbromid in einem etherischen Lösungsmittel wie THF um und unterwirft das erhaltene neue 3-(1-Hydroxy-but-3-in-1-yl)-2,4,4-trimethyl-cyclohex-2-en-1-on-oxim (VIII) einer Partialhydrierung. Auch das Propinylmagnesiumhalogenid verwendet man in mindestens 1-molarem Überschuß, vorteilhaft in Mengen von 2 bis 3 mol pro mol II. Hierbei sind andere übliche Umsetzungen mit Propinylmetallverbindungen vom Typ

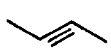

Metall anwendbar. Genannt seien beispielsweise Umsetzungen mit Propinyllithium und Propinylnatrium. Auch hierbei gelingt die Umsetzung mit überraschend hoher Selektivität.

Die Partialhydrierung der Dreifachbindung in dem erhaltenen Alkohol zu dem Alkohol der Formel II gelingt mit hoher Selektivität und einer Ausbeute von 98% der Theorie beispeilsweise an einem mit einem teilweise mit Zink oder Blei vergifteten Palladiumkatalysator auf $CaCO_3$ bei 20 bis 25°C unter Normaldruck. Geeignete Katalysatoren werden beispielsweise in der deutschen Patentschrift 1 115 238 beschrieben.

Zur Überführung von II in den Alkohol IIIc setzt man II in an sich bekannter Weise mit Aceton zu dem neuen 4-(2,6,6-Trimethyl-3-oximido-cyclohex-1-en-1-yl)-but-3-en-2-on (VII), welches dann zu IIIc reduziert wird. Man arbeitet hierbei im allgemeinen mit einem größen Acetonüberschuß, d.h. mit etwa 10 bis 35, vorzugsweise 20 bis 30 mol pro mol II. Als alkalischen Katalysator verwendet man mit Vorteil wäßrige Kalilauge in einer Menge von 0,5 bis 3,5, vorzugsweise 1,5 bis 3 mol pro mol II in Form einer etwa 3 bis 20 %igen wäßrigen Lösung oder alkoholische Alkalialkoholatlösung. Die Reaktionstemperatur beträgt im allgemeinen 20°C bis Siedetemperatur.

Die Reduktion des Ketons VII zu dem Alkohol der Formel IIIc erfolgt auch in an sich bekannter Weise, beispielsweise mit Aluminiumisopropylat in Isopropanol oder Natriumborhydrid in Ethanol.

Die Überführung von II in den Alkohol der Formel IIId erfolgt analog der Überführung von II in den Alkohol IIIa, nur, daß man anstelle eines Allylmagnesiumhalogenids ein Propylmagnesiumhalogenid bzw. eine andere Metall-propyl-verbindung verwendet. Das dabei in 99 %iger Ausbeute anfallende Rohprodukt kann leicht durch Säulenchromatographie an Kieselgel mit Cyclohexan/Essigester als Laufmittel gereinigt werden.

Zur Überführung von II in den Alkohol IIIe setzt man II zunächst wie oben beschrieben mit einem Überschuß an Aceton zu dem neuen 4-(2,6,6-Trimethyl-3-oximido-cyclohex-1-en-1-yl)-3-buten-2-on um,

welches dann mit mindestens einem molaren Überschuß an einem Methylmagnesiumhalogenid analog der oben beschriebenen Weise zu dem neuen Alkohol IIIe umgesetzt werden kann. Aus IIIe kann dann durch gleichzeitige Abspaltung der Oximgruppe und Wasser in der oben beschriebenen Weise ein neuer Riechstoff der Megastigman-Reihe, das 3-(3-Methyl-buta-1,3-dien-1-yl)-2,4,4-trimethyl-cyclohex-2-en-1-on), mit einer interessanten fruchtigen, blumigen und holzigen Duftnote erhalten werden.

Die neuen Alkohole der Formel III sowie die neuen Verbindungen eröffnen einen neuen Weg zur Synthese von wertvollen Duft- und Aromastoffen der Megastigman-Reihe, bei dem man ausgehend vom Citral in nur 4 oder 5 einfachen Reaktionsstufen zu den gewünschten Verbindungen gelangen kann.

## Beispiel 1
### Herstellung von Megastigma-5,7,9-trien-4-on

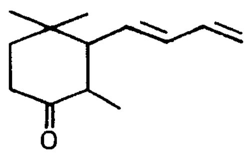

a) Herstellung von 3-(1-Hydroxy-but-3-en-1-yl)-2,4,4-trimethyl-cyclohex-2-en-1-on-oxim (IIIa)

Zu 97,2 g (4 mol) Magnesium und 100 ml absolutem Tetrahydrofuran (THF) wurden 306 g (4 mol) Allylchlorid in 2 l abs. THF getropft. (Die Grignardreaktion wurde durch eine Spur Jod und 1 ml reines Allylchlorid gestartet.) Nach vollendeter Zugabe wurde 1 Stunde bei 35 bis 40°C nachgerührt.

Zu der erhaltenen Lösung von Allylmagnesiumchlorid wurde bei Raumtemperatur eine Lösung von 181 g (1 mol) 1-Formyl-2,6,6-trimethyl-3-oximido-cyclohex-1-en (II) in 200 ml THF getropft. Anschließend wurde 1 h bei Raumtemperatur nachgerührt, dann unterhalb von 10°C 400 ml $H_2O$ zugetropft, 15 min nachgerührt, der gebildete Niederschlag abgesaugt und der Filterrückstand 3 mal mit je 250 ml $CHCl_3$ nachgewaschen. Die vereinigten Filtrate wurden bei 20 mbar eingeengt und der kristalline Rückstand (207 g; Rohausbeute 93%) aus 70 ml Cyclohexan umkristallisiert.

Man erhielt 190 g IIIa vom Schmelzpunkt F = 106—111°C. Das entspricht einer Ausbeute von 85% der Theorie.

IR (Film):
- 3 460 $cm^{-1}$, 3 270 $cm^{-1}$ } OH
- (3 360 $cm^{-1}$ — 2 780 $cm^{-1}$ OH)
- 1 640 $cm^{-1}$ C=C

$^1H$—NMR ($CDCl_3$/270 MHz) δ:
- 9,90 ppm, sbr., 1H, OH
- 5,92 ppm, m, 1H, CH-3′
- 5,20 ppm, "t", 2H
- 4,56 ppm, d,d 1H
- 2,04 ppm, s, 3H, $CH_3$ an C-2
- 2,00 ppm, s(br), 1H, OH
- 1,58 ppm, m, 2H, $CH_2$-6
- 1,19 ppm, s, 3H; 1,09 ppm, s, 3H } $CH_3$ an C-4

$^{13}C$—NMR ($CDCl_3$/50 MHz) δ:
- 158,05 ppm C-1
- 150,95 ppm C-3
- 135,49 ppm C-3′
- 127,28 ppm C-2
- 117,82 ppm C-4′
- 70,87 ppm C-1′
- 41,17 ppm C-2′
- 36,97 ppm C-5′
- 35,28 ppm C-4
- 27,62 ppm; 26,82 ppm } $CH_3$ an C-4
- 19,23 ppm C-6
- 14,28 ppm $CH_3$ an C-2

b) Umsetzung mit 30 %iger Schwefelsäure

111,5 g (0,5 mol) eines gemäß Beispiel 1a erhaltenen IIIa wurden zusammen mit 856 g 30 %iger

wäßriger Schwefelsäure 4 Stunden unter Rückfluß zum Sieden erhitzt. Anschließend wurde das Reaktionsgemisch 6 mal mit Chloroform extrahiert, die vereinigten Extrakte 3 mal mit Wasser und einmal mit $NaHCO_3$-Lösung gewaschen und mit wasserfreiem Natriumsulfat getrocknet und bei einem Druck von 20 mbar abdestilliert. Man erhielt 96 g eines Rohprodukts, das nach gaschromatographischer Analyse (GC) zu ca. 85% aus dem gewünschten Megastigma-5,7,9-trien-4-on (Ausb. 86% d.Th.) bestand. Das Produkt kann durch fraktionierte Destillation gereinigt werden.

Geruchsnote: süß, fruchtig, blumig, teeartig, holzig.

Beispiel 2

a) Darstellung von 3-(1-Hydroxy-but-2-in-1-yl)-2,4,4-trimethyl-cyclohex-2-en-1-on-oxim (VIII)

In 2 l (3 mol) einer 1,5 molaren Lösung von Methylmagnesiumchlorid in Tetrahydrofuran wurden bei 20 bis 25°C im Verlauf von 5 h 300 g (7,5 mol) Propin eingeleitet. Anschließend wurde eine Stunde bei Raumtemperatur nachgerührt. Zu der erhaltenen Lösung von Propinylmagnesiumchlorid tropfte man die Lösung von 181 g (1 mol) 1-Formyl-2,6,6-trimethyl-3-oximido-cyclohexen in 800 ml THF. Das Reaktionsgemisch wurde noch 1 h bei Raumtemperatur nachgerührt und dann unterhalb von 10°C tropfenweise mit 300 ml $H_2O$ versetzt. Der gebildete Niederschlag wurde abgesaugt und 3 mal mit je 500 ml Chloroform gewaschen. Die vereinigten Filtrate wurden bei 20 mbar eingeengt. Der hieraus gebildete kristalline Rückstand (210 g Rohausbeute = 94% d.Th.) wurde aus 1 l Essigsäureethylester umkristallisiert. Man erhielt so 190 g VIII vom Schmelzpunkt F = 162—165°C. Das entspricht einer Ausbeute von 86% der Theorie.

IR (KBr-Preßling):

- 3450 $cm^{-1}$ OH
- (2600—3640 $cm^{-1}$ OH)
- 2210 $cm^{-1}$ C≡C
- 1640 $cm^{-1}$ C=C

$^1H$—NMR ($CDCl_3$/270 MHz) δ:

- 9,90 ppm S, 1H, OH
- 5,27 ppm, S, 1H, CH-1′
- 4,25 ppm, S, 1H, OH
- 2,58 ppm, m, 2H, $CH_2$-6
- 2,06 ppm, s, 3H, $CH_3$-4′
- 1,80 ppm, s, 3H, $CH_3$ an C-2
- 1,54 ppm, t (J~7 Hz), 2H, $CH_2$-5
- 1,20 ppm, s, 3H } $CH_3$ an C-4
- 1,14 ppm, s, 3H }

$^{13}C$—NMR Aceton/90,52 MHz):

- 157,32 ppm C-1
- 148,64 ppm C-3
- 129,26 ppm C-2
- 81,49 ppm C-2′
- 80,50 ppm C-3′
- 60,38 ppm C-1′
- 37,93 ppm C-5
- 35,66 ppm C-4
- 27,83 ppm $CH_3$ an C-4
- 26,87 ppm
- 19,59 ppm C-6
- 14,14 ppm $CH_3$ an C-2
- 3,37 ppm C-4′

b) Darstellung von 3-(1-Hydroxy-cis-but-2-en-1-yl)-2,4,4-trimethyl-cyclohex-2-en-1-on-oxim (IIIb)

93 g (0,42 mol) eines gemäß Beispiele 2a erhaltenen Oxims VIII wurden in 1 l Aceton gelöst und über 10 g Palladium auf $CaCO_3$ (zinkvergiftet) in 2 Stunden bei 20 bis 25°C und Normaldruck hydriert ($H_2$-Aufnahme 10,4 l).

Der Kontakt wurde abfiltriert und das Filtrat bei 20 mbar eingeengt.

Das Rohprodukt (93 g; Rohausbeute = 99 % d.Th.) wurde aus 1 l Cyclohexan umkristallisiert.

Ausbeute 92 g (98% der Theorie).

Fp. 103—108°C.

IR (KBr-Preßling):

- 3260 $cm^{-1}$ OH
- 1652 $cm^{-1}$ } C=C
- 1608 $cm^{-1}$ }
- 1590 $cm^{-1}$ C=NOH

$^1H$—NMR ($CDCl_3$/270 MHz):

10,00 ppm, S(br), 1H, OH
5,83 ppm, d,d ($J_1$~$J_2$~11 Hz), 1H, CH-2'
5,67 ppm, d,q ($J_1$~11 Hz, $J_2$~7 Hz), 1H, CH-3'
5,34 ppm, d, (J~11 Hz), 1H, CH-1'
2,06 ppm, s, 3H, $CH_3$ an C-2
1,79 ppm, d,d ($J_1$~1,5 Hz, $J_2$~7Hz), 3H, $CH_3$-4'
1,58 ppm, "t" (J~7 Hz), 2H, $CH_2$-5
1,21 ppm, s, 3H }
1,04 ppm, s, 3H } $CH_3$ an C-4

$^{13}C$—NMR ($CDCl_3$/90,52 MHz) δ:

158,32 ppm C-1
151,62 ppm C-3
131,81 ppm C-2'
128,55 ppm C-3'
127,72 ppm C-2
66,57 ppm C-1'
36,89 ppm C-5
35,35 ppm C-4
27,66 ppm }
26,71 ppm } $CH_3$ an C-4
19,29 ppm C-6
14,39 ppm C-4'
13,33 ppm $CH_3$ an C-2

c) Das erhaltene IIIb wurde analog Beispiel 1b in Megastigma-5,7,9-trien-4-on überführt.

Beispiel 3

a) Herstellung von 3-(1-Hydroxy-butyl)-2,4,4-trimethylcyclohex-2-en-1-on-oxim (IIId)

Aus 24,3 g Magnesium (1 mol) in 50 ml THF und 148 g (1,2 mol) Propylbromid in 500 ml THF-abs. wurde eine Lösung von Propylmagnesiumbromid in THF hergestellt. Zu der erhaltenen Propylmagnesiumbromid-Lösung wurde tropfenweise bei Raumtemperatur die Lösung von 45,25 g (0,35 mol) II in 300 ml THF addiert, das Gemisch 1 Stunde bei Raumtemperatur nachgerührt, dann tropfenweise unterhalb von 10°C 100 ml Wasser dazugegeben und filtriert. Die erhaltenen Niederschläge wurden 3 mal mit je 100 ml $CHCl_3$ gewaschen und die vereinigten Filtrate bei 20 mbar eingeengt. Man erhielt so 101 g eines Rohproduktes, welches durch präparative Säulenchromatographie gereinigt wurde (Cyclohexan:Essigester = 5:1).

Aus 10 g Rohprodukt wurden an 500 g $SiO_2$ 7,8 g des gewünschten IIId erhalten. Daraus errechnet sich eine Ausbeute von 99% der Theorie.

$^{13}C$—NMR ($CDCl_3$/90,52 MHz) δ:

158,12 ppm C-1
152,41 ppm C-3
126,65 ppm C-2
71,43 ppm C-1'
38,66 ppm C-2'
36,86 ppm C-5
35,27 ppm C-4
27,63 ppm }
26,75 ppm } $CH_3$ an C-4
20,20 ppm C-6
19,23 ppm C-3'
14,27 ppm C-4'
13,95 ppm $CH_3$ an C-2

$^1H$—NMR ($CDCl_3$/80 MHz) δ:

9,03 ppm "s(br.)", 1H, NOH
4,49 ppm, m, 1H, CH-1'
2,01 ppm, s, 3H, $CH_3$ an C-2,
1,15 ppm, s, 3H }
1,08 ppm, s, 3H } $CH_3$ an C-4
0,96 ppm, t, (J~7Hz), $CH_3$-4'

b) Herstellung von Megastigma-5,7-dien-4-on

56 g des gemäß Beispiel 3a erhaltenen Rohproduktes wurden zusammen mit 430 g 30 %iger wäßriger

$H_2SO_4$, 7,5 h unter Rückfluß zum Sieden erhitzt. Das Reaktionsgemisch wurde 3 mal mit jeweils 100 ml Diethylether extrahiert. Die vereinigten Etherextrakte wurden mit Wasser neutral-gewaschen, mit wasserfreiem Natriumsulfat getrocknet und bei 20 mbar eingeengt. Es verblieben 45 g eines Rückstandes der über eine Brücke destilliert wurde.

Ausbeute: 34 g.

Megastigma-5,7-dien-4-on wurde durch präparative Säulenchromatographie an Kieselgel (Cyclohexan/Essigester = 10:1) gereinigt.

Geruchsnote: süßlich, puderartig, würzig, cedernholzartig, schwach krautig, im Nachgeruch fruchtig).

IR (Film)
1663 $cm^{-1}$ C=O
1590 $cm^{-1}$ C=C

$^{13}C$—NMR ($CDCl_3$/90,52 MHz) δ:
199,44 ppm C-4
161,52 ppm C-6
139,44 ppm C-8
129,62 ppm C-5
125,24 ppm C-7
37,34 ppm C-2
35,47 ppm C-1
34,34 ppm C-3
27,36 ppm $CH_3$ an C-1
26,45 ppm C-9
13,53 ppm $CH_3$ an C-5
13,42 ppm C-10

$^1H$—NMR ($CDCl_3$/80 MHz) δ:
6,00 ppm, d, (J~16 Hz), 1H, CH-7
5,63 ppm, d,t (J~16 Hz, $J_2$~6 Hz) 1H, CH-8
2.47 ppm, t (J~7 Hz) 2H, $CH_2$-3
2,20 ppm, d,q ($J_1$~6 Hz, $J_2$~7 Hz) 2H, $CH_2$-9
1,85 ppm, t (J~7 Hz), 2H, $CH_2$-2
1,84 ppm, s, 3H, $CH_3$-13
1,13 ppm, s, 6H, $CH_3$-11,12
1,06 ppm, t (J~7Hz) 3H, $CH_3$-10

## Beispiel 4

Herstellung von 3-(3-Methyl-buta-1,3-dien-1-yl)-2,4,4-trimethylcyclohex-2-en-1-on

a) Herstellung von 4-(2,6,6-Trimethyl-3-oximido-cyclohex-1-enyl)-but-3-en-2-on (VII)

272 g (1,5 mol) II wurden zusammen mit 2,7 l Aceton und 1,1 l einer 5 %igen wäßrigen Kalilauge 5 Stunden unter Rückfluß zum Sieden erhitzt.

Anschließend ließ man das Reaktionsgemisch auf Raumtemperatur abkühlen, neutralisierte mit Eisessig und destillierte das überschüssige Aceton bei 20 mbar ab.

Der 2-phasige Rückstand wurde 3 mal mit je 500 ml $CHCl_3$ extrahiert. Die vereinigten Extrakte wurden 2 mal mit je 500 ml $H_2O$ gewaschen und mit wasserfreiem Natriumsulfat getrocknet. Das Lösungsmittel wurde bei 20 mbar abdestilliert und der erhaltene Rückstand 3 Stunden bei 60°C getrocknet. Man erhielt 366 g rohes VII, das aus 900 ml Methyl-t-Butylether umkristallisiert wurde.

Ausbeute 255 g (77% der Theorie).

Fp. 98—103°C.

IR (KBr-Pressling):
3250 $cm^{-1}$ OH
1660 $cm^{-1}$ C=O
1617 $cm^{-1}$ } C=C
1605 $cm^{-1}$ }

$^1H$—NMR (80 MHz/$CDCl_3$) δ:
7,28 ppm, d (J~16 Hz), 1H, H an C-4
6,14 ppm, d (J~16 Hz), 1H, H an C-3
2,70 ppm, t (J~7Hz), 2H, H an C-4'
2,33 ppm, s, 3H, $CH_3$-1
1,90 ppm, s, 3H, $CH_3$ an C-2'
1,63 ppm, t (J~7Hz), 2H, H an C-5'
1.11 ppm, s, 6H, $CH_3$ an C-6.

$^{13}C$—NMR ($MHz/CDCl_3$) δ:

198,04 ppm, C-2
157,01 ppm, C-3
146,59 ppm, C-1′
141,81 ppm, C-4
133,28 ppm, C-3
128,10 ppm, C-2′
36,14 ppm, C-5′
34,83 ppm, C-6′
27,54 ppm, $CH_3$ an C-6′, C-1
19,41 ppm, C-4′
14,80 ppm, $CH_3$ an C-2′

b) Herstellung von 3-(3-Methyl-3-hydroxy-but-1-enyl)-2,4,4-trimethylcyclohex-2-en-1-on-oxim (IIIe)

Zu 2,2 l einer 1,5 molaren Lösung von Methylmagnesiumchlorid in THF abs. (3,3 mol) wurde unterhalb von 20°C eine Lösung von 246 g (1,1 mol) des gemäß Beispiel 4a erhaltenen Produktes in 1 l THF zugetropft. Man rührte 1 h bei Raumtemperatur nach und fügte unterhalb von 10°C 330 ml $H_2O$ dazu. Anschließend rührte man noch 10 min. nach, saugte die Niederschläge ab und wusch den Filterrückstand 3 mal mit je 250 ml $CHCl_3$. Die vereinigten Filtrate wurden bei 20 mbar eingeengt. Man erhielt 265 g Rohprodukt, das aus 1,3 l Cyclohexan umkristallisiert wurde.

Ausbeute 233 g (98% der Theorie).

Fp. 93—101°C.

IR (KBr-Preßling):

3264 $cm^{-1}$ OH
1598 $cm^{-1}$ C=C

$^1H$—NMR ($CDCl_3$/200 MHz) δ:

6,17 ppm, d } AB-Spektrum (JAB~16,7 Hz) 1H, CH-1′
5,66 ppm, d } AB-Spektrum (JAB~16,7 Hz) 1H, CH-2′
2,70 ppm, t (J~7Hz,), 2H, $CH_2$-6
1,90 ppm, S, 3H, $CH_3$ an C-2
1,63 ppm, t (J~7Hz), 2H, $CH_2$-5
1,43 ppm, S, 6H, $CH_3$-4′ und $CH_3$ an C-3′
1,36 ppm, S, 2H, OH
1,06 ppm, S, 6H, $CH_3$ an C-4

$^{13}C$—NMR ($CDCl_3$/90,52 MHz) δ:

157,45 ppm C-1
149,22 ppm C-3
143,04 ppm C-2′
124,23 ppm C-2
123,24 ppm C-1′
71,20 ppm C-3′
35,89 ppm C-5
34,75 ppm C-4
29,87 ppm $CH_3$-4′ und $CH_3$ an C-3′
27,38 ppm $CH_3$ an C-4
19,51 ppm C-6
14,70 ppm $CH_3$ an C-2

c) Herstellung von 3-(3-Methyl-buta-1,3-dien-1-yl)-2,4,4-trimethyl-cyclohex-2-en-1-on

59,3 g (0,25 mol) des gemäß Beispiel 4b erhaltenen Alkohols IIIe wurden zusammen mit 295 g 30 %iger $H_2SO_4$ 60 min unter Rückflüß zum Sieden erhitzt.

Nach Abkühlen wurde das Reaktionsgemisch 3 mal mit je 100 ml Diethylether extrahiert. Die vereinigten Extrakte wurden mit Wasser neutral gewaschen und mit $Na_2SO_4$ getrocknet. Das Lösungsmittel wurde bei 20 mbar abdestilliert. Man erhielt 47,2 g Rohprodukt (93% Rohausbeute), das über eine Brücke destilliert wurde.

Siedepunkt Kp = 83—84°C bei 0,05 mbar.

Geruchsnote: fruchtig, blumig, Holz Note.

IR (Film):

1670 $cm^{-1}$ C=O unges.
1633 $cm^{-1}$ C=C
1608 $cm^{-1}$ C=C

$^{13}C$—NMR ($CDCl_3$/90,52 MHz) δ:

199,09 ppm C-1
160,85 ppm C-3
141,54 ppm C-3′
138,73 ppm C-2′
130,12 ppm C-2
125,41 ppm C-1′
118,42 ppm C-4′
37,50 ppm C-5
35,71 ppm C-4
34,34 ppm C-6
27,55 ppm $CH_3$ an C-4
18,23 ppm $CH_3$ an C-3′
13,56 ppm $CH_3$ an C-2

$^1H$—NMR ($CDCl_3$/200 MHz) δ:

6,34 ppm, d } 2H, ($J_{AB}$~16 Hz), CH-1′,2′
6,21 ppm, d }
5,06 ppm, s } 2H, CH-4′
5,02 ppm, s }
2,44 ppm, t (J~7 Hz), 2H, $CH_2$-6
1,86 ppm, s, 3H, $CH_3$ an C-2
1,78 ppm, t (J~7 Hz), 2H, $CH_2$-5
1,75 ppm, s, 3H, $CH_3$ an C-3′
1,13 ppm, s, 6H, $CH_3$ an C-4

Beispiel 5

a) Darstellung von 3-(3-Hydroxy-but-1-enyl)-2,4,4-trimethyl-cyclohex-2-en-1-on-oxim

22,1 g (0,1 mol) 4-(2,6,6-Trimethyl-3-oximido-cyclohex-1-enyl)-but-3-en-2-on wurden zusammen mit 100 ml Isopropanol und 20,4 g (0,1 mol) Aluminiumdiisopropylat erhitzt. Über eine 50 cm lange Kolonne ($V_2A$-Netze) wurde unterhalb von 80°C, das sich bildende Aceton und danach 50 ml Isopropanol abdestilliert.

Der Rückstand wurde auf ein Gemisch aus 200 g Eis und 100 ml Wasser gegeben, mit 15 ml konz. Schwefelsäure angesäuert und in 100 ml Diethylether aufgenommen. Die Wasserphase wurde noch 4 mal mit je 25 ml Diethylether extrahiert. Die vereinigten Etherphasen wurden 3 mal mit je 50 ml Wasser sowie 1 mal mit 25 ml 10 %iger $NaHCO_3$-Lösung gewaschen und über wasserfreiem Natriumsulfat getrocknet. Der Ether wurde bei 20 mbar abdestilliert. Man erhielt 23,3 g rohes 3-(3-Hydroxy-but-1-enyl)-2,4,4-trimethyl-cyclohex-2-en-1-on-oxim als Rückstand, welcher durch Umkristallisation aus 25 ml Essigsäureethylester und 5 ml Cyclohexan gereinigt wurde. Hierbei wurden 13,4 g eines nach DC reinen Produktes vom Schmelzpunkt F = 92—95°C erhalten, entsprechend 80% der Theorie. Aus der Mutterlauge ließen sich weitere 8,8 g eines gemäß DC Leicht verunreinigten Produktes gewinnen.

b) Darstellung von Megastigma-5,7,9-trien-4-on

27 g (0,12 mol) des gemäß a) erhaltenen reinen 3-(3-Hydroxy-but-1-enyl)-2,4,4-trimethyl-cyclohex-2-en-1-on-oxims wurden zusammen mit 270 g 30 %iger Schwefelsäure einer Wasserdampfdestillation unterzogen (Badtemperatur 200—250°C). Während der Destillation wurden 2500 ml Wasser langsam zugetropft.

Das Destillat (2,5 l) wurde 5 mal mit jeweils 100 ml Diethylether extrahiert. Die vereinigten Etherextrakte wurden mit wasserfreiem Natriumsulfat getrocknet und der Ether bei 20 mbar abdestilliert. Man erhielt 15,1 g Rückstand, der nach Gaschromatogramm zu 81,3% aus Megastigma-5,7,9-trien-4-on bestand.

Beispiel 6

a) Darstellung von 3-(3-Hydroxy-but-1-enyl)-2,4,4-trimethyl-cyclohex-2-en-1-on-oxim

In eine Lösung von 22,1 g (0,1 mol) 4-(2,6,6-Trimethyl-3-oximido-cyclohex-1-enyl)-but-3-en-2-on in 200 ml Ethanol wurden unter Eiskühlung innerhalb einer halben Stunde 1,9 g Natriumborhydrid portionsweise eingetragen. Es wurde 3 Stunden bei 0 bis 5°C nachgerührt und das Ethanol bei 20 mbar abdestilliert.

Der Rückstand wurde in 200 ml 5 %iger Essigsäure aufgenommen. Man extrahierte 5 mal mit jeweils 100 ml Chloroform.

Die vereinigten Extrakte wurden je einmal mit 50 ml Wasser und 50 ml gesättigter Natriumhydrogencarbonatlösung gewaschen, über wasserfreiem Natriumsulfat getrocknet und bei 20 mbar eingeengt. Es resultieren 10,2 g rohes 3-(3-Hydroxy-but-1-enyl)-2,4,4-trimethyl-cyclohex-2-en-1-on-oxim, das mit dem aus Beispiel 5a identisch war.

b) Das erhaltene Produkt wurde analog Beispiel 5b in Megastigma-5,7,9-trien-4-on überführt.

**Patentansprüche**

1. Verfahren zur Herstellung von Verbindungen der Megastigman-Reihe der allgemeinen Formel I

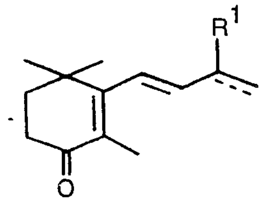

(I),

in der $R^1$ für Wasserstoff oder Methyl steht, und die gestrichelte Linie für eine weitere C—C-Bindung stehen kann, dadurch gekennzeichhnet, daß man

A) das neue 1-Formyl-2,6,6-trimethyl-3-oximido-cyclohex-1-en der Formel II

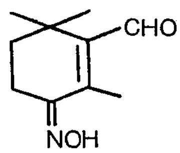

(II)

in an sich bekannter Weise durch Grignardreaktion mit Allylmagnesiumchlorid, Allylmagnesiumbromide, Propylmagnesiumchlorid oder Propylmagnesiumbromid oder durch Grignardreaktion mit Propinylmagnesiumchlorid oder Propinylmagnesiumbromid und anschließende Partialhydrierung der Dreifachbindung der Seitenkette oder aber durch Umsetzen mit Aceton in Gegenwart alkalischer Katalysatoren und anschließende Reduktion der Carbonylgruppe der Seitenkette zur Hydroxylgruppe oder anschließende Grignardreaktion mit Methylmagnesiumchlorid oder Methylmagnesiumbromid in einen neuen Alkohol der allgemeinen Formel III

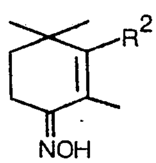

(III),

in der $R^2$ für einen der Reste

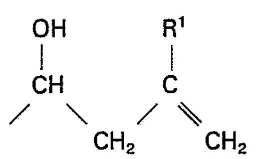 ; 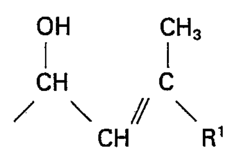 ;

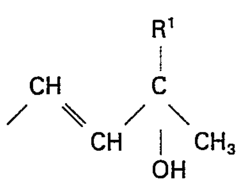 oder 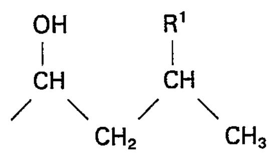

steht, überführt und

B) den erhaltenen Alkohol der Formel III in Gegenwart von Wasser und einer schwerflüchtigen starken Säure auf Temperaturen von 90 bis 120°C erhitzt.

2. Verfahren zur Herstellung von Verbindungen der allgemeinen Formel I

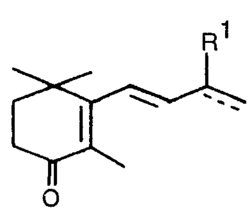

(I),

in der $R^1$ für H steht und die gestrichelte Linie eine weitere C—C-Bindung bedeutet, gemäß Anspruch 1, dadurch gekennzeichnet, daß man in Reaktionsschritt A

a) das 1-Formyl -2,6,6-trimethyl- 3-oximido-cyclohex-1-en der Formel II durch Grignardreaktion mit Allylmagnesiumchlorid oder Allylmagnesiumbromid in einen Alkohol der Formel IIIa

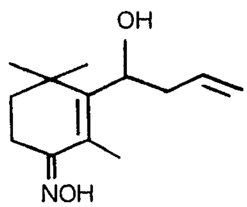

(IIIa)

überführt oder

b) II durch Grignard-Reaktion mit Propinylmagnesiumchlorid oder Propinylmagnesiumbromid zunächst in den neuen Alkohol der Formel VIII

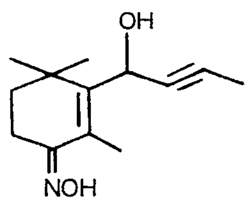

(VIII)

und durch anschließende Partialhydrierung der Dreifachbindung der Seitenkette in einen Alkohol der Formel IIIb

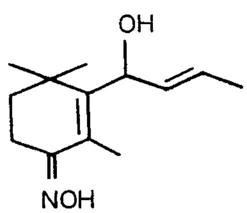

(IIIb)

überführt oder aber

c) II durch Umsetzen mit Aceton in Gegenwart alkalischer Katalysatoren zunächst in das neue Keton der Formel VIII

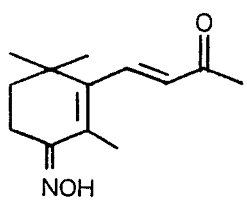

(VII)

und durch anschließende Reduktion der Carbonylgruppe der Seitenkette zur Hydroxylgruppe in den Alkohol der Formel IIIc

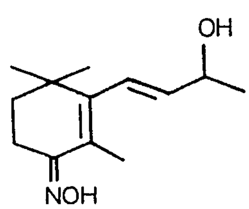

(IIIc)

überführt.

3. Verfahren zur Herstellung von Verbindungen der allgemeinen Formel I

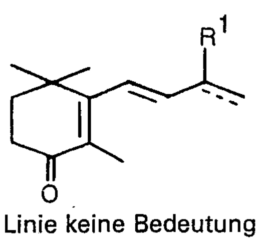

(I),

in der $R^1$ für H steht und die gestrichelte Linie keine Bedeutung hat,

gemäß Anspruch 1, dadurch gekennzeichnet, daß man im Reaktionsschritt A das 1-Formyl-2,6,6-trimethyl-3-oximido-cyclohex-1-en der Formel II durch Grignardreaktion mit Propylmagnesiumchlorid oder Propylmagnesiumbromid in den Alkohol der Formel IIId

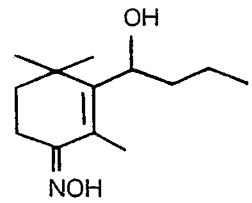

(IIId)

überführt.

4. Verfahren zur Herstellung von Verbindungen der allgemeinen Formel I

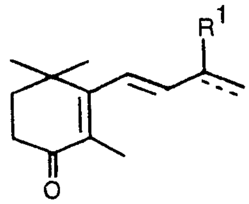

(I),

in der R[1] für $—CH_3$ steht und die gestrichelte Linie eine C—C-Bindung bedeutet, gemäß Anspruch 1, dadurch gekennzeichnet, daß man im Reaktionsschritt A das 1-Formyl-2,6,6-trimethyl-3-oximido-cyclohex-1-en der Formel II durch Umsetzen mit Aceton in Gegenwart alkalischer Katalysatoren zunächst in das neue Keton der Formel VII

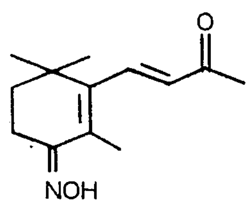

(VII)

und durch anschließende Grignardreaktion mit Methylmagnesiumchlorid oder Methylmagnesiumbromid in den neuen Alkohol der Formel IIIe

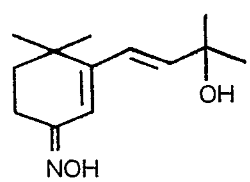

(IIIe)

überführt.

5. Verfahren zur Herstellung von Verbindungen der allgemeinen Formel I

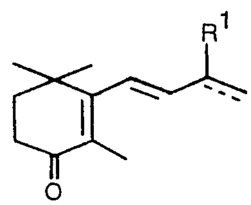

(I),

gemäß Anspruch 1, dadurch gekennzeichnet, daß man im Reaktionsschritt B den Alkohol der Formel III in Gegenwart von 20 bis 40 %iger wäßriger Schwefelsäure auf Temperaturen von 90 bis 120°C erhitzt.

6. Durch die 2,6,6-Trimethyl-3-oximido-1-cyclohexen-1-yl-Gruppe substituierte Alkohole der allgemeinen Formel III

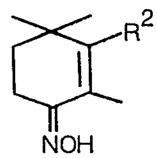

(III),

in der $R^2$ für einen der folgenden Reste steht:

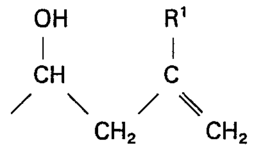 ; 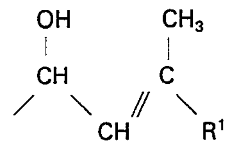 ;

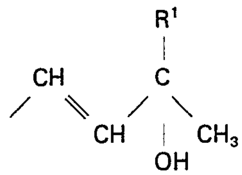 oder 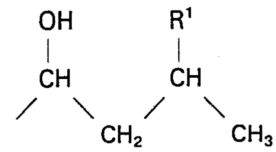

7. 3-(1-Hydroxy-but-3-en-1-yl)-2,4,4-trimethyl-cyclohex-2-en-1-on-oxim (IIIa).
8. 3-(1-Hydroxy-cis-but-2-en-1-yl)-2,4,4-trimethyl-cyclohex-2-en-1-on-oxim (IIIb).
9. 3-(3-Hydroxy-but-1-en-1-yl)-2,4,4-trimethyl-cyclohex-2-en-1-on-oxim (IIIc).
10. 3-(1-Hydroxy-butyl)-2,4,4-trimethyl-cyclohex-2-en-1-on-oxim (IIId).
11. 3-(3-Hydroxy-3-methyl-but-1-en-1-yl)-2,4,4-trimethyl-cyclohex-2-en-1-on-oxim (IIIe).
12. 2-(3-Oxo-but-1-en-1-yl)-2,4,4-trimethyl-cyclohex-2-en-1-on-oxim (VII).
13. 3-(1-Hydroxy-but-2-in-1-yl)-2,4,4-trimethyl-cyclohex-2-en-1-on-oxim (VIII).

**Revendications**

1. Procédé pour la préparation de composés de la série des mégastigmanes de formule générale I

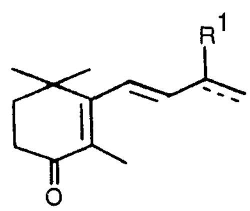

(I),

dans laquelle $R^1$ est mis pour un atome d'hydrogène ou un radical méthyle et la ligne en trait interrompu peut étre mise pour une autre liaison C—C, caracterisé en ce que:

A) on transforme le nouveau 1-formyl-2,6,6-triméthyl-3-oximido-cyclohexène-1 de formule II

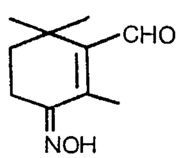

(II)

de façon connue en soi par réaction de Grignard avec le chlorure d'allyl-magnésium, le bromure d'allylmagńesium, le chlorure de propyl-magnésium ou le bromure de propyl-magnésium, ou par réaction de Grignard avec le chlorure de propinyl-magnésium ou le bromure de propinyl-magnésium suivie d'hydrogenation partielle de la triple liaison de la chaine latérale, ou encore par réaction avec l'acétone en présence de catalyseurs alcalins, suivie de réduction du groupe carbonyle de la chaîne latérale en groupe hydroxyle ou suivie de réaction de Grignard avec le chlorure de méthyl-magnésium ou le bromure de méthyl-magnésium, en un alcool nouveau de formule générale III

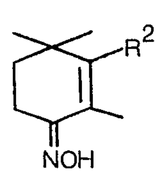

(III),

dans laquelle $R^2$ est mis pour l'un des radicaux

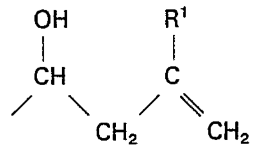 ; 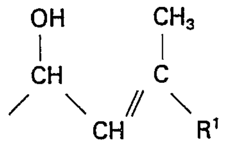 ;

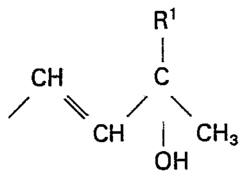 ou 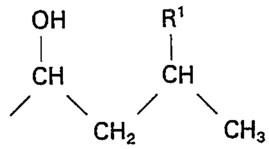

et

B) on chauffe a une température de 90 à 120°C l'alcool de formule III obtenu, en présence d'eau et d'un acide fort de faible volatilité.

2. Procédé pour la préparation de composés de formule générale I

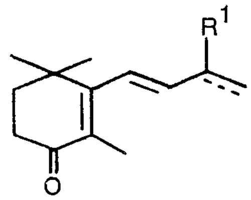

(I),

dans laquelle $R^1$ est mis pour un atome d'hydrogène ou un radical méthyle et la ligne en trait interrompu peut être mise pour une autre liaison C—C, caractérisé en ce que, dans la phase réactionnelle A,

a) on transforme le 1-formyl-2,6,6-triméthyl-3-oximido-cyclohexène-1 de formule II, par réaction de Grignard avec le chlorure d'allyl-magnésium ou le bromure d'allyl-magnésium en un alcool de formule IIIa

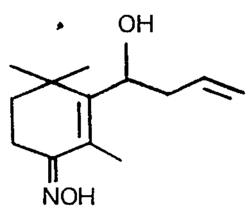

(IIIa)

ou

b) on transforme II, par réaction de Grignard avec le chlorure de propinyl-magnésium ou le bromure de propinyl-magnésium, tout d'abord en l'alcool nouveau de formule VIII

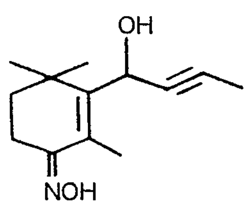

(VIII)

et, par hydrogénation partielle subséquente de la triple liaison de la chaîne latérale, en un alcool de formule IIIb

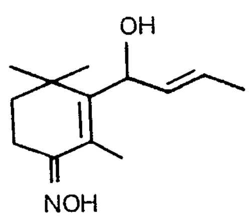

(IIIb)

ou encore

c) on transforme II, par réaction avec l'acétone en présence de catalyseurs alcalins, tout d'abord en la cétone nouvelle de formule VII

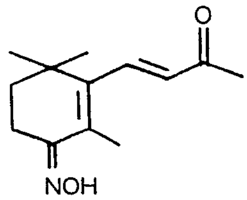

(VII)

et, par réduction subséquente du groupe carbonyle de la chaîne latérale en groupe hydroxyle, en l'alcool de formule IIIc

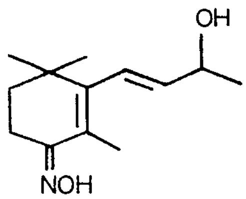

(IIIc)

3. Procédé selon la revendication 1 pour la préparation de composés de formule générale I

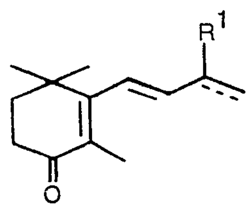

(I),

dans laquelle $R^1$ est mis pour H et la ligne en trait interrompu n'a pas de signification, caractérisé en ce que dans la phase réactionnelle A, on transforme le 1-formyl-2,6,6-triméthyl-3-oximido-cyclohexéne-1 de formule II, par reaction de Grignard avec le chlorure de propylmagnésium ou le bromure de propylmagnésium, an l'alcool de formule IIId

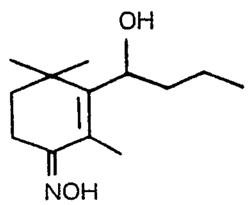

(IIId)

4. Procédé selon la revendication 1 pour la préparation de composés de formule générale I

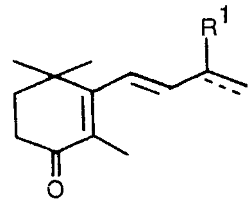

(I),

dans laquelle $R^1$ est mis pur $—CH_3$ et la ligne en trait interrompu représente une liaison C—C, caractérisé en ce que, dans la phase réactionnelle A, on transforme le 1-formyl-2,6,6-triméthyl-3-oximido-cyclohexène-1 de formule II, par reaction avec l'acétone en présence de catalyseurs alcalins, tout d'abord en la cétone nouvelle de formule VII

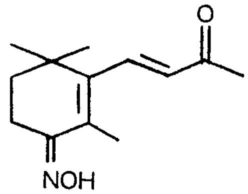

(VII)

et, par réaction de Grignard subséquente avec le chlorure de méthyl-magnésium ou le bromure de méthyl-magnèsium, en l'alcool nouveau de formule IIIe

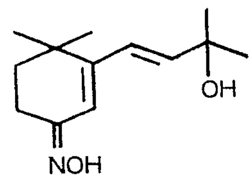

(IIIe)

5. Procédé selon la revendication 1 pour la préparation de composés de formule générale I

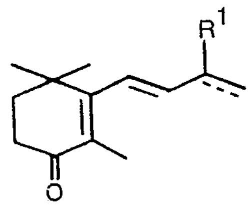

(I),

caracterisé en ce que dans la phase réactionnelle B, on chauffe l'alcool de formule III à une température de 90 à 120°C en présence d'acide sulfurique aqueux à 20—40%.

6. Alcools substitués par le groupe 2,6,6-triméthyl-3-oximido-1-cyclohexényle-1, de formule générale III

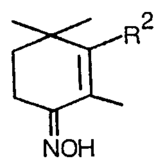

(III),

dans laquelle $R^2$ est mis pour l'un des radicaux suivants

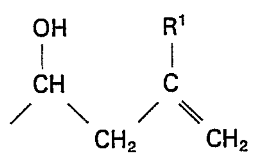 ; 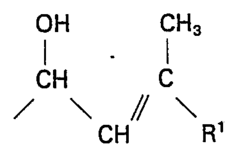 ;

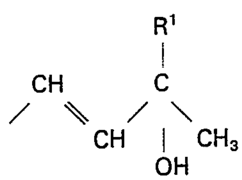 ou 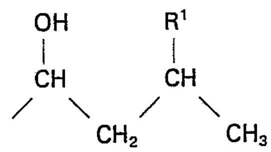

7. 3-(1-hydroxy-but-3-ène-1-yl)-2,4,4-triméthyl-cyclohex-2-ène-1-one-oxime (IIIa).
8. 3-(1-hydroxy-cis-but-2-ène-1-yl)-2,4,4,-triméthyl-cyclohex-2-ène-1-one-oxime (IIIb).
9. 3-(3-hydroxy-but-1-ène-1-yl)-2,4,4-triméthyl-cyclohex-2-ène-1-one-oxime (IIIc).
10. 3-(1-hydroxy-butyl)-2,4,4-triméthyl-cyclohex-2-ène-1-one-oxime (IIId).
11. 3-(3-hydroxy-3-méthyl-but-1-éne-1-yl)-2,4,4-triméthyl-cyclohex-2-ène-1-one-oxime (IIIe).
12. 2-(3-oxo--but-1-éne-1-yl)-2,4,4-triméthyl-cyclohex-2-ène-1-one-oxime (VII).
13. 3-(1-hydroxy-but-2-yne-1-yl)-2,4,4-triméthyl-cyclohex-2-ène-1-one-oxime (VIII).

**Claims**

1. A process for the preparation of a compound of the magastigmane series of the formula I

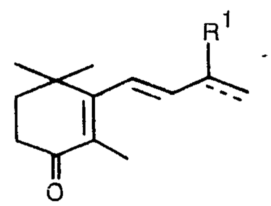

(I),

where $R^1$ is hydrogen or methyl and the broken line can represent a further C—C bond, wherein

A) the novel 1-formyl-2,6,6-trimethyl-3-oximidocyclohex-1-ene of the formula II

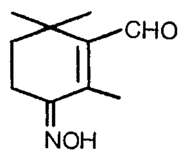

(II)

is converted in a conventional manner by Grignard reaction with allymagnesium chloride, allylmagnesium bromide, proplymagnesium chloride, or propylmagnesium bromide or by Grignard reaction with propynylmagnesium chloride or propynylmagnesium bromide and subseqeuent partial hydrogenation of the triple bond in the side chain or, however, by reaction with acetone in the presence of an alkaline catalyst and subsequent reduction of the carbonyl group in the side chain to a hydroxyl group or subsequent Grignard reaction with methylmagnesium chloride or methylmagnesium bromide to a novel alcohol of the formula III

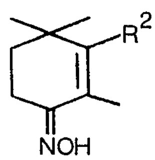

(III),

wherein $R^2$ is

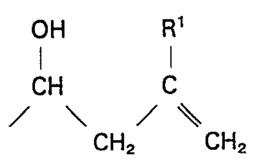 ; 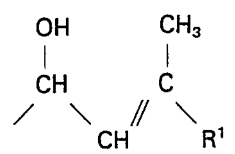 ;

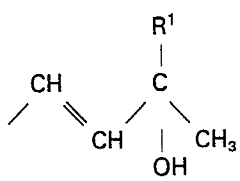 or 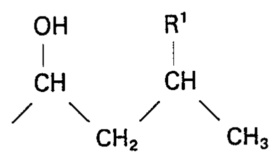

and

B) the resulting alcohol of the formula III is heated at from 90 to 120°C, in the presence of water and of a sparingly volatile strong acid.

2. A process for the prepration of a compound of the formula I

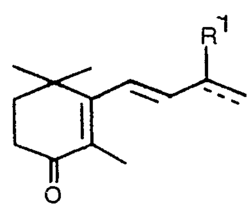

(I),

where $R^1$ is H and the broken line is a further C—C bond, as claimed in claim 1, wherein, in step A,

a) 1-formyl-2,6,6-trimethyl-3-oximidocyclohex-1-ene of the formula II is converted to the alcohol of the formula IIIa

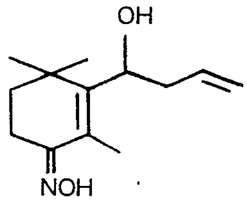

(IIIa)

by means of a Grignard reaction with allylmagnesium chloride or alkylmagnesium bromide, or

b) II is first converted to the novel alcohol of the formula VIII

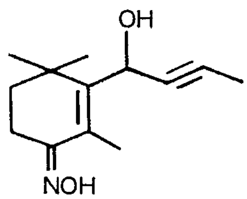

(VIII)

by means of a Grignard reaction with propynylmagnesium chloride or propynylmagnesium bromide, and the product is then converted to the alcohol of the formula IIIb

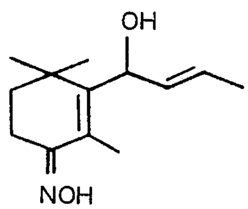

(IIIb)

by partial hydrogenation of the triple bond in the side chain, or

c) II is first reacted with acetone in the presence of an alkaline catalyst to give the novel ketone of the formula VII

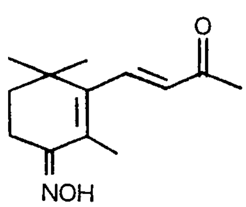

(VII)

and this is converted to the alcohol of the formula IIIc

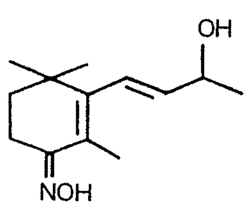

(IIIc)

by reducing the carbonyl group in the side chain to a hydroxyl group.

3. A process for the preparation of a compound of the formula I

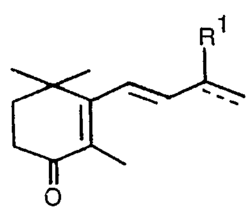

(I),

wherein $R^1$ is H and the broken line has no significance, as claimed in claim 1, wherein, in step A, 1-formyl-2,6,6-trimethyl-3-oximidocyclohex-1-ene of the formula II is converted to the alcohol of the formula IIId

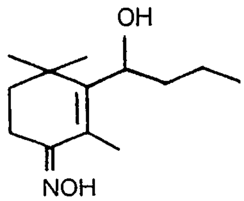

(IIId)

by means of a Grignard reaction with propylmagnesium chloride or propylmagnesium bromide.

4. A process for the preparation of a compound of the formula I

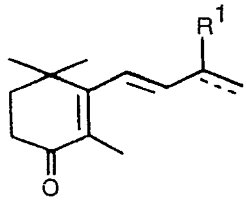

(I),

where $R^1$ is $-CH_3$ and the broken line is a C—C bond, as claimed in claim 1, wherein, in step A, 1-formyl-2,6,6-trimethyl-3-oximidocyclohex-1-ene of the formula II is first converted to the novel ketone of the formula VII

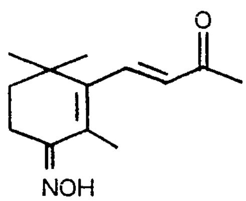

(VII)

by reaction with acetone in the presence of an alkaline catalyst, and the product is subjected to a Grignard reaction with methylmagnesium chloride or methylmagnesium bromide to give the novel alcohol of the formula IIIe

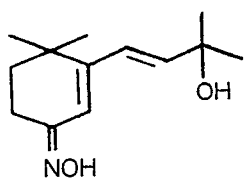

(IIIe)

5. A process for the preparation of a compound of the formula I

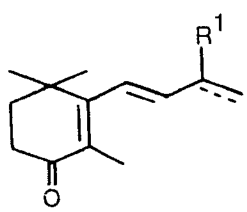

(I),

as claimed in claim 1, wherein, in step B, the alcohol of the formula III is heated at from 90 to 120°C in the presence of 20—40% strength aqueous sulfuric acid.

6. A 2,6,6-trimethyl-3-oximidocyclohex-1-en-1-yl-substituted alcohol of the formula III

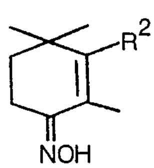

(III),

wherein $R^2$ is

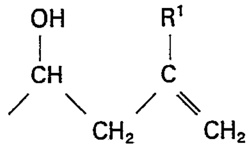 ; 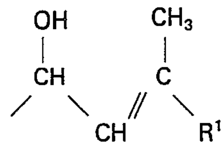 ;

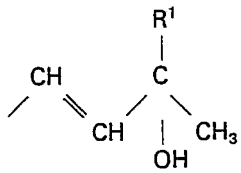 or 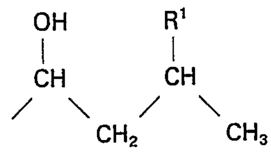

7. 3-(1-Hydroxybut-3-en-1-yl)-2,4,4-trimethylcyclohex-2-en-1-one oxime (IIIa).
8. 3-(1-Hydroxy-cis-but-2-en-1-yl)-2,4,4-trimethylcyclohex-2-en-1-one oxime (IIIb).
9. 3-(3-Hydroxybut-1-en-1-yl)-2,4,4-trimethylcyclohex-2-en-1-one oxime (IIIc).
10. 3-(1-Hydxroxybutyl)-2,4,4-trimethylcyclohex-2-en-1-one oxime (IIId).
11. 3-(3-Hydroxy-3-methylbut-1-en-1-yl)-2,4,4-trimethylcyclohex-2-en-1-one oxime (IIIe).
12. 2-(3-Oxobut-1-en-1-yl)-2,4,4-trimethylcyclohex-2-en-1-one oxime (VII).
13. 3-(1-Hydroxybut-3-yn-1-yl)-2,4,4-trimethylcyclohex-2-en-1-one oxime (VIII).